(19) Europäisches Patentamt
European Patent Office
Office européen des brevets

(11) **EP 3 167 927 B1**

(12) **EUROPEAN PATENT SPECIFICATION**

(45) Date of publication and mention
of the grant of the patent:
**12.06.2019  Bulletin 2019/24**

(51) Int Cl.:
***A61M 21/00*** *(2006.01)*

(21) Application number: **15193790.1**

(22) Date of filing: **10.11.2015**

(54) **SYSTEM AND METHOD FOR REDUCING MOTION SICKNESS OF A VEHICLE PASSENGER**

SYSTEM UND VERFAHREN ZUR REDUZIERUNG DER REISEKRANKHEIT EINES FAHRZEUGINSASSEN

SYSTÈME ET PROCÉDÉ POUR RÉDUIRE LA CINÉTOSE D'UN PASSAGER DE VÉHICULE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**17.05.2017  Bulletin 2017/20**

(73) Proprietor: **Bayerische Motoren Werke Aktiengesellschaft
80809 München (DE)**

(72) Inventors:
• **Hofmann, Marc
  Tokyo, Tokyo 106-0032 (JP)**
• **Yamashita, Yusaku
  Kanagawa, Tokyo 2480013 (JP)**
• **Maki, Sugimoto
  Kanagawa, Tokyo 223-8522 (JP)**
• **Masahiko, Inami
  Kanagawa, Tokyo 233-8521 (JP)**
• **Nakamura, Fumihiko
  Yokohama-city, Tokyo 223 (JP)**
• **Pruneau, Maxime
  Kanagawa, Tokyo 223-8522 (JP)**

(56) References cited:
GB-A- 2 515 616          US-A1- 2012 016 431
US-A1- 2012 203 309      US-A1- 2013 090 704
US-A1- 2014 127 666

## Description

The present invention relates to a system for reducing motion sickness of a vehicle passenger.

Motion sickness is also known as kinetosis or travel sickness. Motion sickness can occur to passengers of any vehicle. Depending on the specific kind of vehicle, motion sickness is sometimes referred to as car sickness, seasickness or air sickness. Symptoms of motion sickness can include dizziness, fatigue and nausea. It is believed that one cause for motion sickness is a disagreement between visually perceived movement and the vestibular system's sense of movement.

For example, motion sickness can occur when a passenger reads a book during car travel. Looking at the book, the passenger's eyes do hardly perceive any motion, whereas the vestibular system senses the motion, in particular any acceleration, that the passenger's body, in particular the passenger's head, is exposed to. Modern vehicles often exhibit displays on which a vehicle's passenger can watch films or other information during travel. Keeping the eyes on such a display can cause kinetosis in a similar way as reading a book.

Motion sickness is particularly unpleasant in highly automated driving situations. In such situations, the vehicle controls the driving operations, while the driver is free to devote himself or herself to other occupations, e.g. watching a film or reading a book.

DE 10 2006 009 566 A1 proposes a method for reducing kinetosis, whereby images from the vehicle's environment are recorded and shown on a vehicle display. In this way, kinetosis of passengers whose outside view is partly or entirely obstructed can be reduced.

DE 10 2007 037 852 A1 proposes a similar method for reducing kinetosis, whereby various visual effects can be shown on the vehicle display. The visual effects are chosen in such a way that they convey an impression corresponding to the current movement of the vehicle.

DE 101 56 219 C1 proposes another method for reducing kinetosis, whereby the visual content of a display of a vehicle is adapted according to measured driving parameters such as direction and magnitude of acceleration, velocity or inclination of the vehicle. The visual content can be enlarged, reduced, moved or rotated. However, this method requires the display to be significantly larger than the actual displayed image.

One means to influence the vestibular system is known as galvanic vestibular stimulation (GVS), whereby specific electric signals are transmitted to a vestibular nerve in the ear. For example, a GVS system can comprise two electrodes that are placed behind a person's ears. A small electric current signal between the electrodes can be transmitted in order to influence the person's vestibular sense.

The article "Galvanic vestibular stimulation: a novel modulatory countermeasure for vestibular-associated movement disorders" by Rizzo-Sierra et al. (DOI: 10.1590/0004-282X20130182) describes GVS as a means to mitigate movement (e.g. gait) disorders that result from kinetosis.

The article "Oculo-vestibular recoupling using galvanic vestibular stimulation to mitigate simulator sickness" by Cevette et al. (DOI: 10.3357/ASEM.3239.2012) proposes to use GVS in order to mitigate simulator sickness, i.e. sickness arising from a mismatch between the visually perceived movement and the vestibular system's sense of standstill as it occurs in static flight or driving simulators.

US 2014/0127666 A1 teaches a GVS stimulation system for alleviating motion-related sickness. The system may be used for simulators, but also for actual vehicles. The system may be configured to match the user's total perceived motion (actual plus induced perceived motion using GVS) with what they are seeing on a display.

It is an objective of the present invention to provide an improved system for use in a vehicle for reducing motion sickness of a vehicle passenger.

The objective is achieved by a system for reducing motion sickness of a vehicle passenger according to the independent claim. Preferred embodiments of the invention are defined in the dependent claims.

According to the invention, a system for reducing motion sickness of a vehicle passenger is provided. The system is suitable for use in or with all vehicles in which passengers can suffer from motion sickness. In particular, the invention is suitable for road vehicles (such as cars, motorcycles, trucks, buses), rail vehicles, watercraft and aircraft. The vehicle passenger may be a driver of the vehicle (i.e., a person in control of the driving operations of the vehicle) or a passive passenger (i.e., a person not in control of the driving operations of the vehicle). If the vehicle is capable of highly automated driving, the vehicle passenger may also be a passive driver, i.e., a person in control of the driving operations of the vehicle who, at times of highly automated driving, is not required to conduct such driving operations. The invention is also suitable for active vehicle simulators such as driving simulators or flight simulators. An active vehicle simulator (for which the term "physical simulator" is sometimes used) is a vehicle simulator that is capable of physically moving the passenger. For example, active vehicle simulators may employ Steward Platforms in order to cause motion of the entire simulator cabin.

The system comprises a physical acceleration determination unit comprising at least one physical acceleration sensor. The physical acceleration determination unit is configured to determine a physical acceleration acting upon the vehicle and / or the passenger.

When the invention is used with active vehicle simulators, the physical acceleration sensor may be a virtual sensor. In such simulators, the physical acceleration is generated by the simulator itself. Therefore, the physical acceleration is inherently known in the active vehicle simulator. The virtual sensor may for example be a software routine implemented in the simulator control system.

In the context of the present invention, acceleration should be understood in a broad sense as any change of a magnitude and / or a direction of a velocity. In particular, the term acceleration includes positive, negative, radial (tangential), lateral and longitudinal acceleration. For example, a vehicle does not exhibit any acceleration (i.e., the acceleration vector has zero magnitude) when the vehicle stands still. The vehicle also does not exhibit any acceleration when the vehicle is in constant motion. For example, an automobile travelling along a straight road at constant velocity does not exhibit acceleration. In practice, however, a vehicle that is in motion constantly experiences at least small magnitudes of acceleration due to frequent changes in velocity and / or direction.

The system according to the invention further comprises a visual acceleration determination unit configured to determine a visual acceleration visually perceived by the passenger. In other words, the visual acceleration is the acceleration that the passenger perceives through his visual sense. The visual acceleration may be determined in physical units (e.g., $m/s^2$). However, the visual acceleration is not an actual (i.e., physical) change of velocity. Rather, it exists in the visual perception of the passenger. The visual acceleration may correspond to the physical acceleration. In particular, this may be the case when a passenger visually observes the vehicle movement (e.g., a driver watching the road). In that case, the physical acceleration and the visual acceleration may be similar or identical. In other cases, the physical acceleration and the visual acceleration may be different. It should be noted that motion sickness typically occurs in these latter cases. For example, a passenger who reads a book while sitting in a moving vehicle may have a very small visual acceleration even though the physical acceleration acting upon the vehicle and acting upon the passenger is non-zero. In a different example, a passenger may sit in a parked vehicle and watch a film of a vehicle driving along a road. In this example, the determined visual acceleration may be non-zero, even though the physical acceleration acting upon the vehicle and the passenger is zero.

The visual acceleration may be determined using a-priori data. For example, when the passenger is observing a non-moving object such as a book or a display of a rear-seat entertainment system, a visual acceleration of zero may be determined. In another example, the passenger may be watching a film on a display. The film may contain sequences of acceleration (e.g., a film showing a car race). The film may further contain metadata for such sequences that contain the acceleration values that are shown in the film, i.e., the visual acceleration. These metadata may be used by the visual acceleration determination unit to determine the visual acceleration.

The system according to the invention further comprises a galvanic vestibular stimulation unit configured to provide an electric stimulation signal to the passenger in order to induce a sensation of virtual acceleration of the passenger.

The system according to the invention further comprises a control unit. It should be noted that the control unit is not necessarily a physical device separate from the other units. For example, the control unit may be a part of the galvanic stimulation unit or a part of another unit present in the vehicle. Moreover, the control unit may be realized by a network of functional units. For example, several electronic control units (ECUs) of the vehicle may jointly form the control unit according to the invention. The functional units then require data connections between them. For example, the functions units (e.g., ECUs) may be connected by a vehicle data bus.

According to the invention, the control unit is configured

- to determine an acceleration mismatch between the physical acceleration and the visual acceleration,
- to determine a mitigation acceleration as a function of the acceleration mismatch such that the mitigation acceleration at least partially mitigates the acceleration mismatch, and
- to determine the electric stimulation signal as a function of the mitigation acceleration such that the induced sensation of virtual acceleration corresponds to the mitigation acceleration.

The inventors have made three observations upon which the invention is based in order to solve the objective. In order to further explain these observations, it should be first noted that although accelerations may be represented as scalar values, it is advantageous to represent them as vectors.

First, the mismatch between the physical acceleration and the visual acceleration is one of the major causes of motion sickness. In a vectorial representation, the mismatch acceleration may be determined as the difference between the (vectorial) physical acceleration and the (vectorial) visual acceleration. For example, when the physical acceleration and the visual acceleration have equal magnitude and equal direction, the (vectorial) mismatch acceleration has a magnitude of zero. In a different example, when the physical acceleration is non-zero and the visual acceleration is zero, the mismatch acceleration is equal to the physical acceleration.

Second, this mismatch of two accelerations that the passenger is exposed to may be partially mitigated (in other words, reduced) by adding a third acceleration, the mitigation acceleration. In the vectorial representation, the mitigation acceleration may be represented by a vector whose magnitude equals the magnitude of the mismatch acceleration but whose direction is opposed to the direction of the mismatch acceleration. In other words, the mitigation acceleration may be chosen such that the (vectorial) sum of the physical acceleration and the mitigation acceleration equals the visual acceleration.

Third, galvanic vestibular stimulation can be used to induce a sensation of virtual acceleration that corresponds to the mitigation acceleration.

In other words, the inventors have found a simple, cost-effective and non-invasive way to mitigate motion sickness. Rather than treating symptoms, the inventions allows to mitigate motion sickness by mitigating the cause of motion sickness.

According to the invention, the at least one physical acceleration sensor comprises an eye tracker unit configured to determine an eye movement of at least one of the passenger's eyes. This embodiment advantageously uses the fact that the vestibular system can cause reflex actions of the ocular muscles. By taking into account the eye movement, the system can therefore work more adaptively and more precisely. The eye tracking unit may be used as the only sensor means for determining the physical acceleration. Alternatively, the eye tracking unit may be used in combination with another physical acceleration sensor in order to obtain more precise estimates of the physical acceleration.

With particular advantage, the physical acceleration determination unit is configured to determine the physical acceleration acting upon the vehicle and / or the passenger from the eye movement by exploiting the vestibule-ocular reflex (VOR), which is a reflex eye movement that elicits eye movement by stimulating the vestibular system. This reflex functions to stabilize images on the retinas during head movement by producing eye movements in the direction opposite to head movement, thus preserving the image on the center of the visual field. For example, when the head moves to the right, the eyes move to the left, and vice versa. The VOR does not depend on visual input. It works even in total darkness or when the eyes are closed. The VOR can cause an eye movement into any direction. In particular, the eye movements that can be determined by the eye tracker may include

- a pitching (tilting) forward or backward and / or
- a swiveling (yawing) left or right and / or
- a pivoting (rolling) to the left side or to the right side.

Accordingly, the eye movement can be used to estimate the direction of the physical acceleration perceived by the passenger in any direction.

In further preferred embodiments, the physical acceleration determination unit is configured to adaptively determine the physical acceleration acting upon the vehicle and / or the passenger from the eye movement. In other words, the function that is used to map the determined eye movement (input of the function) to the physical acceleration (output of the function) can be a variable function. In particular, the function can be adaptively determined to accommodate various passengers or varying states of the same passenger. For example, different passengers may react differently (i.e., with a different eye movement) to acceleration, because the VOR may be differently pronounced for different persons. Also, the same passenger may react differently, because the VOR may be differently pronounced at different times, for example depending on the vigilance of the passenger.

In further preferred embodiments, the control unit and the eye tracker unit form a control loop, wherein the eye movement is a control variable of the control loop. It should be noted that the VOR can be elicited by electric or thermal stimulation of the vestibular organs in the inner ear. Hence, the VOR causes an eye movement in response to galvanic vestibular stimulation. In other words, the eye movement not only reflects the physical acceleration, but it also reflects the sensation of virtual acceleration induced by galvanic vestibular stimulation. The control loop may be a continuous control loop. In other words, the eye movement may be continuously tracked and the control unit may continuously adapt the electrical stimulation signal in order to reach the targeted eye movement. The embodiment yields the advantage that the system continuously ensures that the induced sense of virtual acceleration in fact closes the gap of the acceleration mismatch and thus mitigates the motion sickness.

In preferred embodiments of the invention, the control unit is configured to determine the mitigation acceleration such that a magnitude of the mitigation acceleration equals a magnitude of the acceleration mismatch and such that a direction of the mitigation acceleration is opposite to a direction of the acceleration mismatch. In other words, the mitigation acceleration is determined such that the vectorial sum of the acceleration mismatch and the mitigation acceleration is zero.

Further preferred, the acceleration mismatch may be determined as the vectorial difference of the physical acceleration and the visual acceleration. Then, the vectorial sum of the physical acceleration and the mitigation acceleration equals the visual acceleration. This preferred embodiment can be expressed in equations as follows, where $a_{physical}$ is the physical acceleration, $a_{visual}$ is the visual acceleration, $a_{mismatch}$ is the acceleration mismatch and $a_{mitigation}$ is the mitigation acceleration.

$$a_{mismatch} = a_{physical} - a_{visual}$$

$$a_{mitigation} = -a_{mismatch}$$

$$a_{physical} + a_{mitigation} = a_{visual}$$

As can be seen from the above explanations and equations, the galvanic vestibular stimulation is used to add an induced sense of virtual acceleration to the actually present physical acceleration in order to comply with the visual acceleration. In other words, it could be said that galvanic vestibular stimulation is used to close the gap between the physical acceleration and the visual acceleration.

According to advantageous embodiments the at least one physical acceleration sensor comprises a vehicle mounted acceleration sensor configured to determine the physical acceleration acting upon the vehicle. The vehicle mounted acceleration sensor may be embodied by an inertial measurement unit configured to measure an inertial force of the vehicle. Many modern vehicles do already exhibit an inertial measurement unit (IMU) able to measure a direction and a magnitude of acceleration. The IMU can be used to provide its measurement data, from which the physical acceleration may be derived.

In further preferred embodiments, the at least one physical acceleration sensor comprises a head mounted acceleration sensor mounted to the passenger's head, the head mounted acceleration sensor being configured to determine the physical acceleration acting upon the passenger's head.

Alternatively or additionally, the at least one physical acceleration sensor may comprise a camera configured to determine the physical acceleration acting upon the passenger's head. Video cameras directed at the passenger's (in particular the driver's) head are already frequently used in modern vehicles for various purposes. Using such a camera for the present invention is therefore possible at limited cost. The images filmed by the camera may be further processed in a processing unit (e.g., in the physical acceleration determination unit) in order to derive the physical acceleration acting upon the passenger's head.

The advantage of determining the physical acceleration acting upon the passenger's head by using a head mounted acceleration sensor and / or a camera lies in an increase in precision. The passenger's head is not necessarily exposed to the same acceleration (or inertial) forces as the vehicle or the display. This is because damping elements such as the seat or the passenger's spine located between the vehicle and the passenger can absorb part of the acceleration forces exerted upon the vehicle. In addition, the movement of the head in response to an acceleration of the vehicle also depends on whether and how prepared the passenger is for the acceleration. For example, if a passenger is focused on a book and does not observe the environment of the vehicle, the passenger's head may be moved relatively strongly when the vehicle drives along a curve. In contrast, if the passenger is observing the road ahead of the vehicle, the passenger will be prepared for the upcoming curve. By tensing the neck muscles, the passenger can hold his head in a relatively constant position despite the acceleration forces exerted upon his head.

The same camera may be used to measure movements of the passenger's eyes and the passenger's head. In that case, the same camera can be used to determine the eye movement and the physical acceleration acting upon the passenger's head.

It is understood that the described embodiments may be advantageously combined. In particular, a very useful embodiment of the invention uses measurements from both an IMU and a camera to determine the physical acceleration.

Advantageously, the system may further comprise a portable electronic device with a display for presenting content to the passenger. Such a portable electronic device may be a smartphone or a tablet computer or any other hand-held device. The portable electronic device comprises an acceleration sensor configured to determine the physical acceleration acting upon the personal electronic device. The control unit is configured to determine the acceleration mismatch from the physical acceleration acting upon the personal electronic device. In other words, the portable electronic device can be configured to measure the acceleration acting upon it. This acceleration may comprise two components. First, it includes the physical acceleration acting upon the vehicle (in which the portable electronic device is located). Second, it includes acceleration relative to the vehicle. The latter acceleration is caused by movement of the portable electronic device relative to the vehicle. In particular, this movement originates from hand movement of the passenger's hand that holds the portable electronic device. Depending on the configuration of the acceleration sensor of the portable electronic device, the determined physical acceleration acting upon the personal electronic device may be the total physical acceleration (i.e., the physical acceleration acting upon the vehicle and the acceleration relative to the vehicle) or the acceleration relative to the vehicle only.

This embodiment yields a twofold advantage. One the one hand, knowledge of the physical acceleration acting upon the personal electronic device can be used to increase the precision of determining the physical acceleration acting upon the vehicle and / or the passenger and / or to increase the precision of determining the acceleration mismatch. On the other hand, the movement of the portable electronic device constitutes or contributes to the visual acceleration. Knowledge of the physical acceleration acting upon the personal electronic device can therefore be used to increase the precision of determining the visual acceleration.

It is understood that both the foregoing general description and the following detailed description are exemplary, and are intended to provide further explanation of the invention as claimed. Other advantages and features of the invention will be apparent from the following detailed description of the invention, which describes exemplary embodiments of the invention, taken in conjunction with the accompanying drawing, in which:

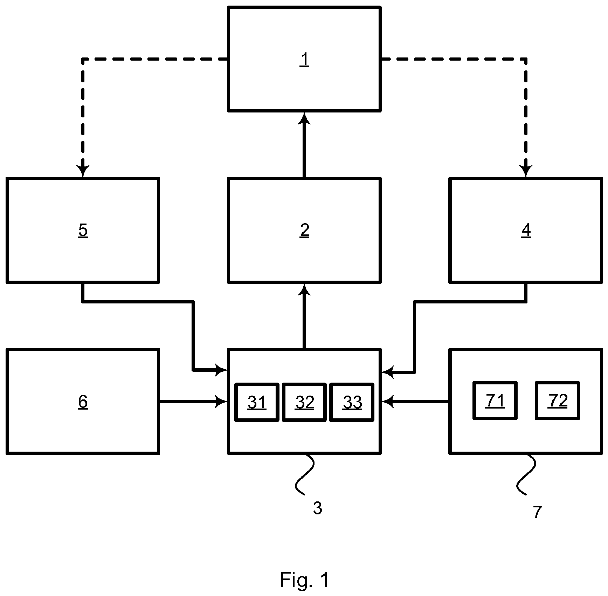

Fig. 1 is a schematic illustration of an exemplary embodiment of the invention.

It is emphasized that the depicted embodiment is an exemplary embodiment of the invention. Combinations of features in this example are not to be understood in a limiting sense. Other embodiments of the invention may exhibit less, more or other features. The extent of the protection and the disclosed subject-matter are determined by the claims and the entire description. It is further noted that the figure depicts a schematic and abstract illustration of the exemplary embodiment. The arrangement of the depicted elements is exemplary and may be different for different embodiments.

Fig. 1 shows a schematic illustration of an exemplary embodiment of the claimed system for reducing motion sickness of a vehicle passenger 1. The depicted system may be used in a road vehicle such as an automobile. The system comprises a galvanic vestibular stimulation unit 2 configured to provide an electric stimulation signal to the passenger 1. The system further comprises a central electronic control unit 3, which may be a central electronic control unit of the vehicle. The control unit 31, the physical acceleration determination unit 32 and the visual acceleration determination unit 33 are comprised by the central electronic control unit 3.

Further depicted in Fig. 1 are a total of four physical acceleration sensors 4, 5, 6, 72. An eye tracker unit 4 is configured to determine the eye movement of the passenger's 1 eyes. The eye tracker unit 4 may be a vehicle camera directed at the head or the eyes of the passenger 1. In this case, the camera may additionally and independently be used as a physical acceleration sensor configured to determine the physical acceleration acting upon the passenger's 1 head. The eye tracker unit 4 may also be a device mounted to the passenger's 1 head. In particular, smart glasses or other optical head-mounted displays comprising a camera may be used as an eye tracker unit 4.

The system further comprises a head mounted acceleration sensor 5 mounted to the head of the passenger 1.

The system further comprises a vehicle inertial measurement unit (IMU) 6. The IMU 6 is used as a vehicle mounted acceleration sensor configured to determine the physical acceleration acting upon the vehicle.

The passenger 1 holds a portable electronic device (PED) 7, which may for example be a smart phone or a tablet computer. The PED 7 comprises a display 71 and an acceleration sensor 72.

The acceleration information determined by each of the physical acceleration sensors 4, 5, 6, 72 can be transferred to the central electronic control unit 3. This data communication is indicated in Fig. 1 by solid arrow lines. The communication links used for this data transfer may be wireline or wireless communication links. For example, the IMU 6 and the central electronic control unit 3 may be connected by a data bus (not shown) of the vehicle. The eye tracker unit 4, the head mounted acceleration sensor 5 and / or the portable electronic device 7 may be connected to the central electronic control unit 3 by a wireless data communication link (e.g., a Bluetooth, WLAN or Zigbee link).

In Fig. 1, dashed arrow lines leading from passenger 1 to head mounted acceleration sensor 5 as well as to eye tracker unit 4 indicate that an acceleration of the passenger's 1 head induces an acceleration of the head mounted acceleration sensor 5 and of the eye tracker unit 4. In contrast, IMU 6 is not directly affected by an acceleration or movement of the passenger's 1 head. The PED 7 may be mounted in a mounting structure of the vehicle, in which case it is not directly affected by an acceleration or movement of the passenger 1.

Although there are four physical acceleration sensors 4, 5, 6, 72 in the example depicted in Fig. 1, one physical acceleration sensor is sufficient to carry out the invention. In other words, any single one of the sensors 4, 5, 6, 72 would be sufficient to carry out the invention. However, the system may operate more effectively and / or more precisely with an increased number of sensors.

The central electronic control unit 3 of the vehicle has a three-fold function. First, it serves as the control unit 31 controlling the galvanic vestibular stimulation unit 2. Second, the central electronic control unit 3 serves as the physical acceleration determination unit 32. Third, the central electronic control unit 3 serves as the visual acceleration determination unit 33.

As explained above, the system for reducing motion sickness of a vehicle passenger 1 according to the invention is particularly useful whenever an acceleration mismatch between the physical acceleration and the visual acceleration is determined.

The visual acceleration determination unit 33 is configured to determine a visual acceleration visually perceived by the passenger 1. If visual acceleration metadata is available within the system, the visual acceleration determination unit 33 may use this metadata to determine the visual acceleration. For example, the vehicle may be a dynamic driving simulator whose physical acceleration is controlled by control unit 3. The dynamic driving simulator may comprise a screen on which a real-time video of the driving situation is shown to the passenger 1. The content shown on the screen may be controlled by control unit 3. The visual acceleration metadata (i.e., the information about the visual acceleration shown on the screen to the passenger 1) may be provided to the visual acceleration determination unit 33. In a different example, passenger 1 may be watching a video on a screen (e.g., on a vehicle display or on the display 71 of PED 7). Visual acceleration metadata of this video may be provided to the visual acceleration determination unit 33. For example, the PED 7 may transmit such metadata to the control unit 3 by means of the wireless (e.g., Bluetooth) data communication link.

If no metadata is available, the visual acceleration determination unit 33 may be configured to determine the visual acceleration visually perceived by the passenger 1 as follows. If the passenger 1 is an active driver of the vehicle (i.e., a driver who actively operates the vehicle), the visual acceleration may be determined to equal the previously determined physical acceleration acting upon the vehicle and / or the passenger 1. The underlying assumption is that an active driver usually observes the road and hence visually perceives the physical acceleration. In this case, no acceleration mismatch between the physical acceleration and the visual acceleration would be determined and the system would not provide an electric stimulation signal to the passenger.

In contrast, if the passenger 1 is a passive driver (i.e., a driver who is currently not actively operating the vehicle, e.g., in phases of autonomous driving) or a passenger other than the driver, the visual acceleration may be determined as zero. The underlying assumption is that a passenger 1 who is not actively driving the vehicle is very likely to watch a static object. For example, a passive driver (or a passenger 1 other than the driver) may look at the display 71 of PED 7 or may read a book.

In the next step, the control unit 31 will determine the acceleration mismatch between the physical acceleration and the visual acceleration. Next, the control unit 31 will determine the mitigation acceleration as a function of the acceleration mismatch such that the mitigation acceleration at least partially mitigates the acceleration mismatch.

In the next step, the control unit 31 will determine the electric stimulation signal as a function of the mitigation acceleration such that the induced sensation of virtual acceleration corresponds to the mitigation acceleration. Specific information about the passenger 1 saved in control unit 3 or accessible by control unit 3 may be used for this purpose. For example, if the system has collected information about how the passenger 1 responded to galvanic vestibular stimulation on previous occasions, it may utilize such information to determine, adapt and / or calibrate the electric stimulation signal.

The control unit 31 and the eye tracker unit 4 may form a control loop, wherein the eye movement is a control variable of the control loop. In that case, information about the eye movement determined by the eye tracker unit 4 is fed back to the control unit 31. The control unit 31 may utilize such information to adapt and / or calibrate the electric stimulation signal such that a desired target eye movement is achieved.

**List of reference signs**

1   Passenger
2   Galvanic vestibular stimulation unit
3   Central electronic control unit
31  Control unit
32  Physical acceleration determination unit
33  Visual acceleration determination unit
4   Eye tracker unit
5   Head mounted acceleration sensor
6   Vehicle inertial measurement unit
7   Portable electronic device (PED)
71  Display

72  Acceleration sensor

**Claims**

1.  System for reducing motion sickness of a vehicle passenger (1), the system comprising

    - a physical acceleration determination unit (32) comprising at least one physical acceleration sensor (4, 5, 6, 72), the physical acceleration determination unit (32) configured to determine a physical acceleration acting upon the vehicle and / or the passenger (1),
    - a visual acceleration determination unit (33) configured to determine a visual acceleration visually perceived by the passenger (1),
    - a galvanic vestibular stimulation unit (2) configured to provide an electric stimulation signal to the passenger (1) in order to induce a sensation of virtual acceleration of the passenger (1) and
    - a control unit (31),

    wherein the control unit (31) is configured

    - to determine an acceleration mismatch between the physical acceleration and the visual acceleration,
    - to determine a mitigation acceleration as a function of the acceleration mismatch such that the mitigation acceleration at least partially mitigates the acceleration mismatch, and
    - to determine the electric stimulation signal as a function of the mitigation acceleration such that the induced sensation of virtual acceleration corresponds to the mitigation acceleration,

    **characterized in that** the at least one physical acceleration sensor (4, 5, 6, 72) comprises an eye tracker unit (4) configured to determine an eye movement of at least one of the passenger's (1) eyes.

2.  System according to claim 1, wherein the physical acceleration determination unit (32) is configured to determine the physical acceleration acting upon the vehicle and / or the passenger from the eye movement by exploiting the vestibule-ocular reflex.

3.  System according to claim 2, wherein the physical acceleration determination unit (32) is configured to adaptively determine the physical acceleration acting upon the vehicle and / or the passenger (1) from the eye movement.

4.  System according to claim 2 or 3, wherein the control unit (31) and the eye tracker unit (4) form a control loop, wherein the eye movement is a control variable of the control loop.

5. System according to any of the preceding claims, wherein the control unit (31) is configured to determine the mitigation acceleration such that a magnitude of the mitigation acceleration equals a magnitude of the acceleration mismatch and such that a direction of the mitigation acceleration is opposite to a direction of the acceleration mismatch.

6. System according to any of the preceding claims, wherein the at least one physical acceleration sensor (4, 5, 6, 72) comprises a vehicle mounted acceleration sensor (6) configured to determine the physical acceleration acting upon the vehicle.

7. System according to any of the preceding claims, wherein the at least one physical acceleration sensor (4, 5, 6, 72) comprises a head mounted acceleration sensor (5) mounted to the passenger's (1) head, the head mounted acceleration sensor (5) being configured to determine the physical acceleration acting upon the passenger's (1) head.

8. System according to any of the preceding claims, wherein the at least one physical acceleration sensor (4, 5, 6, 72) comprises a camera, the camera being configured to determine the physical acceleration acting upon the passenger's (1) head.

9. System according to any of the preceding claims, further comprising a portable electronic device (7) with a display (71) for presenting content to the passenger (1), the portable electronic device (7) comprising an acceleration sensor (72) configured to determine the physical acceleration acting upon the portable electronic device (7), wherein the control unit (31) is configured to determine the acceleration mismatch from the physical acceleration acting upon the portable electronic device (7).

**Patentansprüche**

1. System zum Reduzieren von Reisekrankheit eines Fahrzeuginsassen (1), wobei das System umfasst:

- eine Einheit zur Bestimmung physikalischer Beschleunigung (32), die mindestens einen Sensor physikalischer Beschleunigung (4, 5, 6, 72) umfasst, wobei die Einheit zur Bestimmung physikalischer Beschleunigung (32) so konfiguriert ist, dass sie eine physikalische Beschleunigung bestimmt, die auf das Fahrzeug und/oder den Insassen (1) wirkt,
- eine Einheit zur Bestimmung optischer Beschleunigung (33), die so konfiguriert ist, dass sie eine optische Beschleunigung bestimmt, die vom Insassen (1) optisch wahrgenommen wird,
- eine Einheit zur galvanisch-vestibulären Stimulation (2), die so konfiguriert ist, dass sie ein elektrisches Stimulationssignal für den Insassen (1) bereitstellt, um eine Empfindung virtueller Beschleunigung des Insassen (1) zu induzieren, und
- eine Steuereinheit (31),

wobei die Steuereinheit (31) konfiguriert ist zum:

- Bestimmen eines Beschleunigungsunterschieds zwischen der physikalischen Beschleunigung und der optischen Beschleunigung,
- Bestimmen einer Minderungsbeschleunigung als Funktion des Beschleunigungsunterschieds, derart dass die Minderungsbeschleunigung den Beschleunigungsunterschied wenigstens teilweise mindert, und
- Bestimmen des elektrischen Stimulationssignals als Funktion der Minderungsbeschleunigung, derart dass die induzierte Empfindung virtueller Beschleunigung der Minderungsbeschleunigung entspricht,

**dadurch gekennzeichnet, dass** der mindestens eine Sensor physikalischer Beschleunigung (4, 5, 6, 72) eine Augenverfolgungseinheit (4) umfasst, die zum Bestimmen einer Augenbewegung mindestens eines der Augen des Insassen (1) konfiguriert ist.

2. System nach Anspruch 1, wobei die Einheit zur Bestimmung physikalischer Beschleunigung (32) so konfiguriert ist, dass sie die physikalische Beschleunigung, die auf das Fahrzeug und/oder den Insassen wirkt, aus der Augenbewegung durch Auswerten des vestibulookulären Reflexes bestimmt.

3. System nach Anspruch 2, wobei die Einheit zur Bestimmung physikalischer Beschleunigung (32) so konfiguriert ist, dass sie die physikalische Beschleunigung, die auf das Fahrzeug und/oder den Insassen (1) wirkt, adaptiv aus der Augenbewegung bestimmt.

4. System nach Anspruch 2 oder 3, wobei die Steuereinheit (31) und die Augenverfolgungseinheit (4) eine Regelkreis bilden, wobei die Augenbewegung eine Regelgröße des Regelkreises ist.

5. System nach einem der vorhergehenden Ansprüche, wobei die Steuereinheit (31) so konfiguriert ist, dass sie die Minderungsbeschleunigung derart bestimmt, dass eine Größe der Minderungsbeschleunigung gleich einer Größe des Beschleunigungsunterschieds ist, und derart, dass eine Richtung der Minderungsbeschleunigung entgegengesetzt zur Richtung des Beschleunigungsunterschieds ist.

**6.** System nach einem der vorhergehenden Ansprüche, wobei der mindestens eine Sensor physikalischer Beschleunigung (4, 5, 6, 72) einen an einem Fahrzeug montierten Beschleunigungssensor (6) umfasst, der so konfiguriert ist, dass er die physikalische Beschleunigung bestimmt, die auf das Fahrzeug wirkt.

**7.** System nach einem der vorhergehenden Ansprüche, wobei der mindestens eine Sensor physikalischer Beschleunigung (4, 5, 6, 72) einen auf den Kopf aufsetzbaren Beschleunigungssensor (5) umfasst, der auf den Kopf des Insassen (1) aufgesetzt wird, wobei der auf den Kopf aufsetzbare Beschleunigungssensor (5) so konfiguriert ist, dass er die physikalische Beschleunigung bestimmt, die auf den Kopf des Insassen (1) wirkt.

**8.** System nach einem der vorhergehenden Ansprüche, wobei der mindestens eine Sensor physikalischer Beschleunigung (4, 5, 6, 72) eine Kamera umfasst, die so konfiguriert ist, dass sie die physikalische Beschleunigung bestimmt, die auf den Kopf des Insassen (1) wirkt.

**9.** System nach einem der vorhergehenden Ansprüche, ferner umfassend eine tragbare elektronische Vorrichtung (7) mit einer Anzeige (71) zum Darstellen von Inhalt für den Insassen (1), wobei die tragbare elektronische Vorrichtung (7) einen Beschleunigungssensor (72) umfasst, der so konfiguriert ist, dass er die physikalische Beschleunigung bestimmt, die auf die tragbare elektronische Vorrichtung (7) wirkt, wobei die Steuereinheit (31) so konfiguriert ist, dass sie den Beschleunigungsunterschied aus der physikalischen Beschleunigung bestimmt, die auf die tragbare elektronische Vorrichtung (7) wirkt.

**Revendications**

**1.** Système permettant de réduire le mal des transports d'un passager d'un véhicule (1), ce système comprenant :

- une unité de détermination d'une accélération physique (32) comprenant au moins un capteur d'accélération physique (4, 5, 6, 72), l'unité de détermination d'une accélération physique (32) étant réalisée pour déterminer l'accélération physique agissant sur le véhicule et/ou le passager (1),
- une unité de détermination d'une accélération visuelle (33) réalisée pour déterminer l'accélération visuelle perçue visuellement par le passager (1),
- une unité de stimulation vestibulaire galvanique (2) réalisée pour fournir un signal de stimulation électrique au passager (1) de façon à induire une sensation d'accélération virtuelle de ce passager (1), et
- une unité de commande (31),

cette unité de commande (31) étant réalisée :

- pour déterminer un décalage d'accélération entre l'accélération physique et l'accélération visuelle,
- pour déterminer une accélération d'atténuation en tant que fonction du décalage d'accélération de sorte que l'accélération d'atténuation atténue au moins partiellement le décalage d'accélération, et
- pour déterminer le signal de stimulation électrique en tant que fonction de l'accélération d'atténuation de sorte que la sensation induite d'accélération virtuelle corresponde à l'accélération d'atténuation, **caractérisé en ce que**

le capteur d'accélération physique (4, 5, 6, 72) comporte une unité de suivi oculaire (4) réalisée pour déterminer le mouvement oculaire d'au moins l'un des yeux du passager (1).

**2.** Système conforme à la revendication 1, dans lequel l'unité de détermination de l'accélération physique (32) est réalisée pour déterminer l'accélération physique en agissant sur le véhicule et/ou le passager à partir du mouvement oculaire en exploitant le réflexe vestibulo oculaire.

**3.** Système conforme à la revendication 2, dans lequel l'unité de détermination de l'accélération physique (32) est réalisée pour déterminer de façon adaptative l'accélération physique agissant sur le véhicule et/ou le passager (1) à partir du mouvement oculaire.

**4.** Système conforme à la revendication 2 ou 3, dans lequel l'unité de commande (31) et l'unité de suivi oculaire (4) forment une boucle de commande, le mouvement oculaire étant la commande variable de la boucle de commande.

**5.** Système conforme à l'une quelconque des revendications précédentes, dans lequel l'unité de commande (31) est réalisée pour déterminer l'accélération d'atténuation de sorte que l'amplitude de l'accélération d'atténuation soit égale à l'amplitude du décalage d'accélération et de sorte que la direction de l'accélération d'atténuation soit opposée à la direction du décalage d'accélération.

**6.** Système conforme à l'une quelconque des revendications précédentes,

dans lequel le capteur d'accélération physique (4, 5, 6, 72) comporte un capteur d'accélération (6) monté sur le véhicule et réalisé pour déterminer l'accélération physique agissant sur le véhicule.

7.  Système conforme à l'une quelconque des revendications précédentes, dans lequel le capteur d'accélération physique (4, 5, 6, 72) comporte un capteur d'accélération (5) monté sur la tête du passager (1), ce capteur d'accélération de tête (5) étant réalisé pour déterminer l'accélération physique agissant sur la tête du passager (1).

8.  Système conforme à l'une quelconque des revendications précédentes, dans lequel le capteur d'accélération physique (4, 5, 6, 72) comporte une caméra, cette caméra étant réalisée pour déterminer l'accélération physique agissant sur la tête du passager (1).

9.  Système conforme à l'une quelconque des revendications précédentes, comprenant en outre un dispositif électronique portatif (7) équipé d'un affichage (71) permettant de présenter un contenu au passager (1), le dispositif électronique portatif (7) comprenant un capteur d'accélération (72) réalisé pour déterminer l'accélération physique agissant sur le dispositif électronique portatif (7), l'unité de commande (31) étant réalisée pour déterminer le décalage d'accélération à partir de l'accélération physique agissant sur le dispositif électronique portatif (7).

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102006009566 A1
- DE 102007037852 A1
- DE 10156219 C1
- US 20140127666 A1

**Non-patent literature cited in the description**

- **RIZZO-SIERRA et al.** *Galvanic vestibular stimulation: a novel modulatory countermeasure for vestibular-associated movement disorders*
- **CEVETTE et al.** *Oculo-vestibular recoupling using galvanic vestibular stimulation to mitigate simulator sickness*